Europäisches Patentamt

European Patent Office

Office européen des brevets

(19)

(11) Numéro de publication: **0 007 256 B1**

# FASCICULE DE BREVET EUROPEEN

(12)

(45) Date de publication du fascicule du brevet:
02.10.85

(51) Int. Cl.⁴: **A 61 B 17/36**, A 61 F 9/00

(21) Numéro de dépôt: **79400370.7**

(22) Date de dépôt: **07.06.79**

(54) **Appareil de chirurgie ophtalmologique.**

(30) Priorité: **08.06.78 FR 7817111**

(43) Date de publication de la demande:
**23.01.80 Bulletin 80/2**

(45) Mention de la délivrance du brevet:
**02.10.85 Bulletin 85/40**

(84) Etats contractants désignés:
**AT BE CH DE FR GB IT LU NL SE**

(56) Documents cités:
**DE - A - 1 541 165**
**FR - A - 1 500 039**
**FR - A - 1 557 974**
**FR - A - 2 197 614**
**FR - A - 2 285 842**
**US - A - 3 348 547**
**US - A - 3 710 798**
**US - A - 3 750 670**
**US - A - 3 971 382**
**US - A - 4 069 823**

**"OPTICS" par Hecht et Zajac, p. 307**
**GRAND LAROUSSE, Perot**
**Brockhaus: Warenzeichen R tome 20, p. 21**

(73) Titulaire: **Aron-Rosa, Danièle Sylvie, 28 avenue Raphael, F-75016 Paris (FR)**
Titulaire: **Griesemann-Laporte, Michèle Gabrielle Roberte, La Tetoie, F-02310 Nogent l'Artaud (FR)**

(72) Inventeur: **Aron-Rosa, Danièle Sylvie, 28 avenue Raphael, F-75016 Paris (FR)**
Inventeur: **Griesemann-Laporte, Michèle Gabrielle Roberte, La Tetoie, F-02310 Nogent l'Artaud (FR)**

(74) Mandataire: **Casanova, André et al, Cabinet Casanova et Akerman 23 Boulevard de Strasbourg, F-75010 Paris (FR)**

## Description

La présente invention a pour objet un appareil de chirurgie ophtalmologique comprenant au moins une source laser et un procédé pour la mise en œuvre dudit appareil destiné en particulier mais non exclusivement à la chirurgie ophtalmologique.

### Art antérieur

On sait que les sources laser sont maintenant couramment utilisées dans différents domaines de la chirurgie. Des lasers à rubis ont été utilisés pour tenter de pratiquer la coagulation rétinienne entre 1965 et 1968.

Ces traitements avaient pour but la prophylaxie de l'extension d'une déchirure rétinienne périphérique ou encore la limitation des proliférations néo-vasculaires dans le cas de rétinopathie diabétique. Ces lasers mettaient en jeu des impulsions lumineuses de longueur d'onde égale à 6328 Å d'une durée de quelques millisecondes et d'une énergie de quelques centaines de millijoules. Dans ce mode opératoire, le phénomène physique consistait en la transformation d'énergie lumineuse en énergie thermique dans la couche d'épitélium pigmenté. Mais pratiquement l'apparition fréquente de bulles d'explosion et d'hémorragies dans le virtré a conduit à un usage très limité.

Des photo-coagulateurs à laser Argon sont apparus après 1968. Leur intérêt reposait sur leur excellente répétitivité des caractéristiques de l'irradiation, sur leur stabilité et l'excellente absorption du rayonnement vert à 5140 Å par l'épithélium pigmenté et les tissus vascularisés rouges. Ces appareils sont toujours utilisés avec profit et mettent en jeu des puissances allant de 0,1 à 1,5 Watt véhiculées par des impulsions dont la durée varie de $10^{-2}$ à 1 seconde. La coagulation des substances protéiniques pigmentées résulte d'un effet thermique, la brûlure consécutive à cet effet conduisant par l'apparition des tissus cicatriciels subséquents à la formation de »points de soudure rétinienne«. Malheureusement, un tel rayonnement est inefficace sur des tissus blancs sauf mise en jeu de puissances dangereuses pour l'endothélium cornéen. Ce même type de laser a parfois été utilisé pour relâcher, par brûlure du sphincter irien, un myosis serré mettant à profit la pigmentation du tissu irien.

Des tentatives de perforation de l'iris (iridotomie) ont été faites au moyen de ce laser à Argon continu. Les lésions cornéennes d'origine thermique qui en ont résulté on conduit à renoncer à ce type de traitement de certains glaucomes. Des essais d'iridotomie utilisant un laser à colorants: rhodamine 640, cresyl violet etc. émettant dans le domaine visible des impulsions de l'ordre de 100 millijoules en 1 microseconde ont été effectués en 1978. Malheureusement, si les résultats anatomiques se sont révélés satisfaisants, le caractère traumatisant du traitement a conduit à rechercher des méthodes plus douces pour le patient.

On a tenté à partir de 1974 de traiter les glaucomes avec un laser à rubis. Le principe de ce traitement est la réouverture du trabéculum, tissu qui, à l'état pathologique, cesse l'être poreux et obstrue l'évacuation de l'humeur aqueuse par le canal de Schlemn. Des énergies de l'ordre de 200 millijoules véhiculées par des impulsions de 50 nanosecondes ont été focalisées sur des surfaces de trabéculum d'un diamètre de 250 à 500 micromètres. Les perforations ainsi obtenues se rebouchent dans un délai de deux mois maximum par l'apparition de tissus cicatriciels dus aux effets thermiques. En 1975, M. Krasnov a publié des résultats obtenus avec un laser à rubis ou avec un laser au néodyme dans le traitement des cataractes molles et des membranules de cataractes secondaires. En ce qui concerne les cataractes molles, la résorption des masses cristalliniennes dure environ un an après perforation de la capsule antérieure du cristallin par impact laser. Dans tous les cas, une intervention préalable est nécessaire, intervention qui a pour but de faire migrer des pigments colorés de l'iris vers les zones blanches à perforer. La présence de ce pigment coloré est nécessaire à l'efficacité des lasers de ce type.

Le brevet US-A-3 971 383 décrit un procédé et un appareil pour la chirurgie ophtalmologique, comme les étapes de découpe du cristallin et/ou membranes de la pupille avec un faisceau laser devant passer à travers la cornée, la cavité interne de l'oeil et la pupille, et ce faisceau est ensuite focalisé sur la capsule antérieure dur cristallin et/ou de la membrane de la pupille.

Le faisceau laser peut comporter une ou plusieurs impulsions d'une longueur d'onde comprise entre 5300 et 10 600 Å, avec une durée d'impulsion comprise entre $10^{-7}$ et $10^{-12}$ secondes et la puissance de sortie étant comprise entre $10^7$ et $10^9$ watts.

Toutefois dans l'article du »British Journal of Ophtalmology« (1975), 59, p. 96—98, l'inventeur, M. Krasnov reconnaît que son procédé n'est pas valable pour les tissus transparents mais seulement pour les tissus pigmentés (voir p. 97, colonne de gauche, I.26—42).

Contrairement à ce brevet américain, la présente invention propose un procédé et un appareillage pour découper un tissu biologique, y compris un tissu transparent, assurant une chirurgie ophtalmologique indépendante de la nature chimique et de la couleur du tissu.

Dans le document US-A-3 750 670 on utilise un cristal de KDP interne, fonctionnant en convergent et il est bien connu de l'homme du métier que le faisceau doit être au contraire parallèle et que dans ce cas, le cristal de KDP doit être externe, ce qui est également le cas dans la présente demande.

Mais surtout l'appareil décrit dans le brevet US-A-3 750 670 est construit pour pouvoir émettre des radiations soit à 6600 Å pour la limitation des détachements rétiniens soit à 5320 Å pour le traitement des vaisseaux rétiniens chez les diabétiques. Il apparaît donc de manière évidente que cet appareil est destiné à réaliser une photocoagulation rétinienne et ne peut en aucune manière être destiné à de la chirurgie, à une coupure par effet de claquage. L'effet de ce type d'appareil est strictement thermique à but de cautérisation ou coagulation. Il n'entre donc pas dans le cadre de l'appareil selon la présente invention.

### Objet de l'invention

L'objet principal de la présente invention est un appareil permettant de couper des tissus intraoculaires sans ouverture du globe, cette section étant indépendante de la nature chimique et de la couleur du tissu. Il s'agit en fait de réaliser la coupure du tissu sand échauffement de l'endroit coupé de sorte que cette opération ne génère pas de tissu cicatriciel.

Un second objet de la présente invention est un appareil qui permette la sécurité des tissus adjacents.

Pratiquement, les tissus à couper sont:

— les masses blanches d'un cristallin cataracté,
— les membranes transparentes de la capsule cristallinienne,
— les membranules transparentes ou opacifiées qui résultent de l'opération d'une cataracte (cataractes secondaires),
— les brides de condensation de l'humeur vitrée qui, par leur traction provoquent ou maintiennent des déchirures rétiniennes.

Par contre, l'appareil selon l'invention doit éviter tout impact non voulu du rayonnement sur la cornée, le cristallin et la rétine.

Selon la présente invention, l'appareil de chirurgie ophtalmologique est caractérisé en ce qu'il comprend une source laser émettant un flux lumineux de puissance élevée, puissance véhiculée par au moins une impulsion dont la durée est comprise entre 20 et 400 picosecondes.

### Processus physique

Selon l'invention, le niveau élevé de puissance du flux lumineux supérieur à $10^{12}$ W/cm² est le facteur déterminant du processus physique d'interaction entre le rayonnement et la matière, processus qui permet la destruction localisée d'un tissu sans apparition d'effet thermique et sans nécessité de pigmentation de la cible. Ce processus peut être décrit de la façon suivante:

a) ionisation multiphotonique bien que les potentiels d'ionisation des atomes constituant la matière vivante (N, H, C, O) soient tous supérieurs à 10 eV et bien que l'énergie protée par un photon dont la longueur d'onde $\lambda = 1,06$ micromètre ne soit que de 1,18 eV, l'intensité du flux lumineux est assez forte pour permettre l'action simultanée d'un nombre suffisant de photons qui réalisent le passage de l'état électriquement neutre de la matière à la formation d'un nuage l'électrons libres.

b) Absorption électronique d'énergie
Il s'agit de la cession d'énergie cinétique du champ électrique de l'onde lumineuse aux électrons libérés. Par le processus couramment désigné par l'expression »Bremsstrahlung inverse«, les électrons, lors des collisions avec les atomes et les ions, transforment cette énergie cinétique dirigée en énergie thermique électronique et, par un processus collisionnel, en cascade, propagent l'état d'ionisation et d'énergie électronique dans le milieu environnant. A ce stade, le plasma opaque au rayonnement est créé.

c) Propagation d'une onde de conduction électronique. Dans les première centaines de picosecondes qui suivent les radiations et sur un diamètre de l'ordre de 100 micromètres auotur de l'impact, c'est l'onde de conduction électronique qui se propage, cédant peu à peu son énergie aux particules lourdes, à savoir les atomes et les ions.

d) Dans les quelques nanosecondes qui suivent c'est une onde de choc hydrodynamique qui se propage mais avec un amortissement inversement proportionnel au cube de la distance ($1/R^3$) ce qui assure l'effet mécanique de destruction froide.

Le dispositif suivant l'invention permet d'éviter l'apparition de tissus cicatriciels importants et l'élévation de température des tissus transparents traversés mais qui ne sont pas à détruire; il permet la section chirurgicale de tissus, par »claquage optique«, par la concentration d'impulsions lumineuses ultra-brèves d'un laser, au moyen de lentilles de focalisation.

### Description d'un mode de réalisation de l'appareil

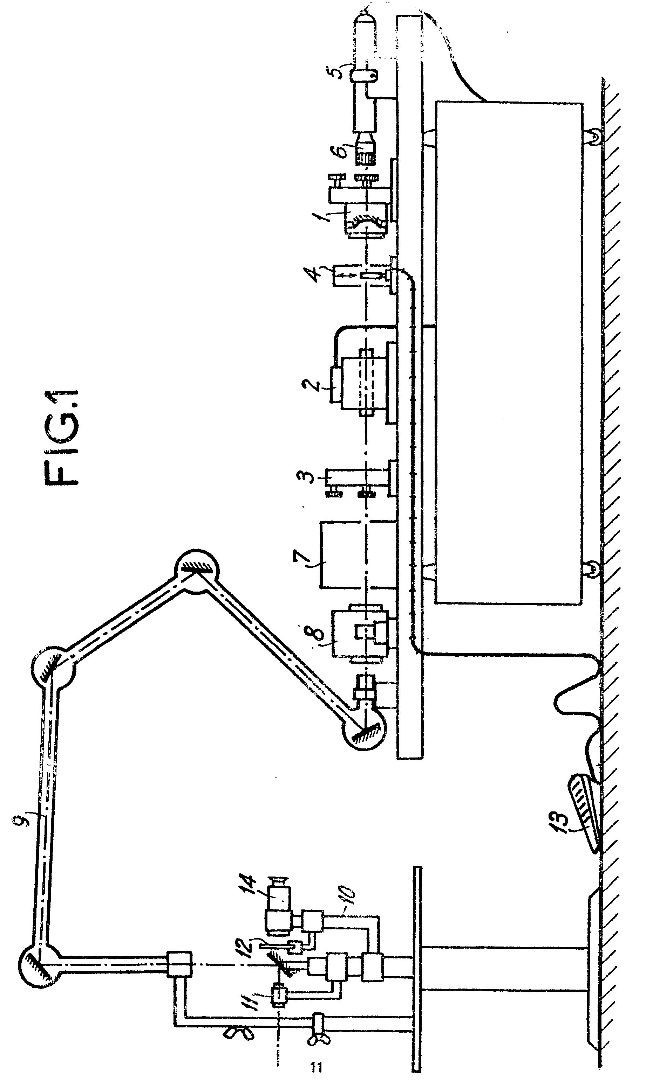

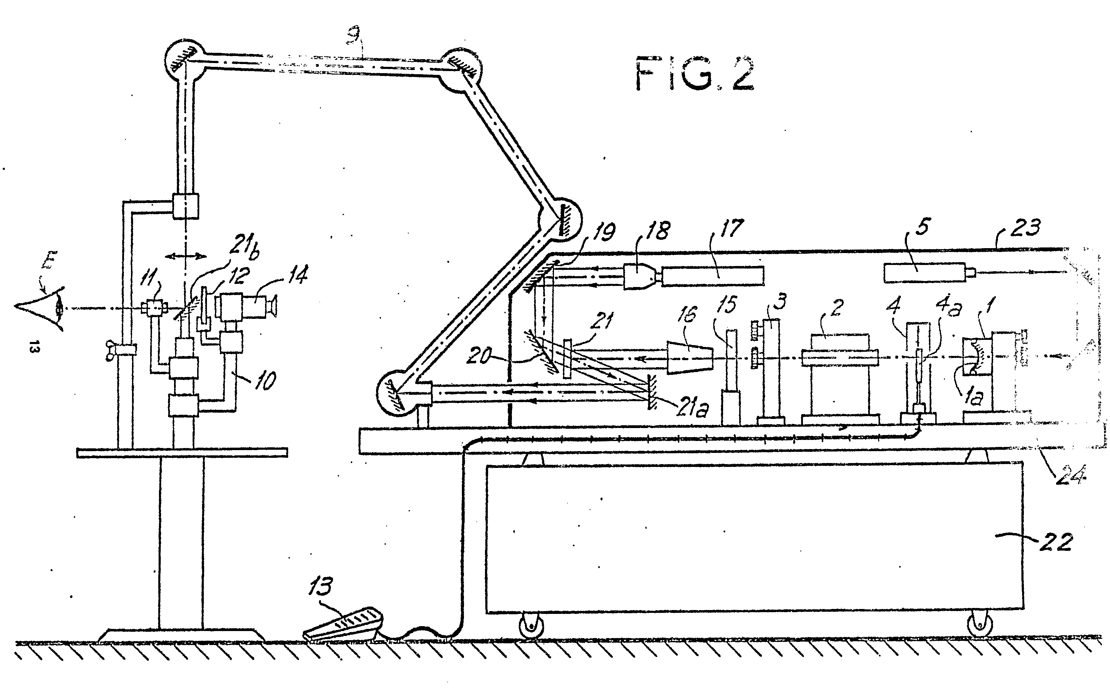

D'autres caractéristiques et avantages de l'invention apparaîtront au cours de la description qui va suivre d'un mode de réalisation particulier en regard des figures 1 et 2 qui représentent un appareil de chirurgie ophtalmologique appelé dans la pratique VITREOTOME ou VITRECTOME et une variante de réalisation.

Sur la figure 1, le dispositif objet de l'invention comporte un oscillateur laser composé de:

— une cuve (1) contenant un miroir et une solution d'absorbant saturable »KODAK 9740 ®«;

— une tête laser (2) contenant un barreau de YAG dopé au Néodyme;

— un étalon de PEROT-FABRY (3), c'est-à-dire une lame composée de substance transparente à faces parallèles d'égal pouvoir réflecteur; ces deux faces constituant un interféromètre d'épaisseur parfaitement maîtrisée. Cet interféromètre utilisé en réflexion comme un miroir de sortie laser a pour effet de supprimer, par interférence destructive, certaines longueurs d'ondes dans la bande spectrale de fluorescence du milieu emissif du laser et, par contrecoup, de contrôler la durée de l'impulsion émise, cet effet étant dû à la relation d'incertitude de Heisenberg. Cet étalon ferme la cavité et permet de choisir la durée des impulsions entre 20 et 400 picosecondes;

— un obturateur (4) à commande électrique et dont le »rideau« est composé d'une lame de verre SCHOTT KG3 ®, absorbante dans l'infra-rouge, mais laissant passer le faisceau visible d'un laser d'alignement (5) Hélium-Néon, d'une puissance de 1 milliwatt et coïncidant avec le faisceau infrarouge du laser opératoire. Un système optique, dit »afocal« (6) permet d'ajuster la divergence de ce faisceau d'alignement de façon à en faire coïncider le point focal avec celui du laser opératoire au point d'opération.

Il peut comporter, en outre, selon les effets thérapeutiques recherchés, un dispositif électro-optique (7) permettant de ne laisser passer qu'une des impulsions du train émis par le laser. Un cristal de KDP (8), convertissant la longueur d'onde d'émission de 10 645 Å en radiation verte à 5322,5 Å, en vue de la réalisation d'une photo-coagulation connue avec le même appareil.

Le faisceau est transporté jusqu'à une lampe à fente d'ophtalmologie classique, par un bras articulé (9) creux, à l'intérieur duquel des miroirs réfléchissent ce faisceau jusqu'à la lampe à fente (10). Celle-ci est munie d'un système optique de focalisation par lentilles (11), qui permet de concentrer le faisceau laser au point opératoire.

Un déclencheur à pédale (13) commande l'ouverture de l'obturateur (4), laissant libres, pour la visée, les mains de l'opérateur. Lorsque l'obturateur est ouvert, le laser à YAG qui est déclenché à une cadence de 1 à 0,3 Hz émet un train d'impulsions qui est utilisé pour la chirurgie; un système électrique interdit l'émission d'un nouveau train par refermeture asservie de l'obturateur (4). Un nouveau coup de pédale est nécessaire pour obtenir un nouveau tir.

La lampe à fente est également munie d'une lame (12) de verre SCHOTT KG3 ®, placée sur l'axe optique de la lunette d'observation (14) qui permet la vision du faisceau d'alignement tout en protégeant les yeux de l'opérateur contre les réflexions possibles du faisceau opératoire.

Fonctionnant avec pour milieu actif un barreau de YAG dopé au NEODYME, le laser (2) émet à une longueur d'onde de 10 645 Å des impulsions de lumière infra-rouge, ces impulsions se présentent sous la forme d'une rafale ou d'un train de 5 à 9 »pics« d'une durée unitaire allant de 20 à 30 picosecondes et séparées de 6 nanosecondes. L'énergie contenue dans ce train de »pics«, à la sortie de l'oscillateur laser est de 10 à 15 millijoules. Ce type d'émission est assuré par le fonctionnement en régime de blocage de modes (mode locking) de l'oscillateur. Le régime de blocage de mode est également connu de l'homme de l'art sous les noms de »synchronisation de phase« ou »mode locking« et recouvre l'émission d'un laser déclenché passivement par l'action d'un absorbant saturable (type KODAK 9740 ® ) qui, ne passant à l'état transparent que sous l'action d'une impulsion de puissance suffisante oblige la cavité laser à n'émettre son énergie que sous la forme d'une série (rafale) d'impulsions d'une durée individuelle égale à celle qui a »ouvert« l'absorbant saturable séparées d'un intervalle égal au temps d'aller-retour entre les miroirs de la cavité, dure sous la forme d'un trains de »pics« ou impulsions. Ce type de fonctionnement est dû à la présence dans la cavité d'une cuve (1) d'absorbant saturable »KODAK« 9740 ® qui fait circuler, en permanence ce produit devant l'un des miroirs (épaisseur de produit traversé; 1 millimètre). Il peut être utile de n'utiliser pour un bombardement qu'une seule impulsion. Dans ce cas, la sélection peut être obtenue par un sélecteur d'impulsions (7) du type de celui fabriqué par la Société française QUANTEL ou la Société britannique J. K. LASERS.

Le faisceau lumineux émis par l'oscillateur laser composé des éléments suivants: tête de laser (2), obturateur (4), étalon de Perot-Fabry (3) et cuve réfléchissante (1), passe par un sélecteur d'impulsions (7), un cristal de KDP (8), puis entre dans un bras articulé (9) creux, aux articulations duquel des miroirs réfléchissent le faisceau en lui conservant ses propriétés de parallélisme et de cohésion spatiale jusqu'à un système optique de focalisation (11).

Le dispositif comprend également un laser hélium-néon (5) de 1 milliwatt qui, muni d'un système afocal (6), permet de modifier la divergence de son faisceau rouge de façon à faire coïncider, au point d'impact, le point focal rouge avec celui de l'infra-rouge.

A l'arrivée au sommet de la lampe à fente (10), un boîtier contient un miroir diélectrique, réfléchissant l'infrarouge et transparent au visible; ce miroir particulier permet:

a) de laisser passer dans l'axe de la lampe à fentes la lumière blanche permettant d'éclairer le champ opératoire à traiter;

b) de limiter la puissance du faisceau rouge de repérage à une valeur inférieure au seuil de danger défini par les normes internationales (réflexion en rouge: 4%);

c) de conserver intégrale l'énergie infrarouge nécessaire au traitement.

Les faisceaux rouge et infrarouge sont focalisés par un doublet (11) achromatique convergent d'une puissance de 10 dioptries, placé sur l'axe de la lampe à fente (10), et à une distance telle que, compte tenu de la réflexion finale, par un miroir, dans une direction horizontale, le point de focalisation soit situé précisément dans le plan de vision nette du biomicroscope.

La sécurité de l'opérateur est assurée contre les réflexions de lumière laser par la présence, dans le biomicroscope d'un filtre fixe (12) composé d'une lame de verre »SCHOTT KG3 ®«, d'une épaisseur de 5 mm, transparente dans le visible et atténuant d'un facteur $10^8$ dans l'infrarouge.

L'énergie lumineuse infrarouge contenue dans chaque tir, au niveau de l'utilisation a été, pour les opérations ci-dessous, d'au minimum 2 millijoules, et d'au maximum 5 millijoules.

Les dimensions des taches focales, réglables par les dispositifs optiques à lentilles ont été de 50 à 100 micromètres de diamètre.

La combinaison de la petitesse des taches focales de l'extrême brièveté des pics lumineux, la modestie des énergies mises en jeu et la forte convergence d'un faisceau de fort diamètre (15 mm) est l'élément déterminant qui a permis de circonscrire très précisément la zone de tissus à détruire et d'annihiler les effets thermiques.

Les puissances élevées mises ainsi en jeu ($10^{12}$ Watts/cm$^2$) se sont avérées être un facteur de sécurité pour la rétine située dans le prolongement de la ligne de tir: en effet, elles assurent la transformation de la cible, même transparente, en un gaz l'électrons et d'ions, plasma dont la densité électronique est supérieure à la densité critique pour laquelle ce gaz devient opaque au rayonnement (pour une longueur d'onde de 10 645 Å, cette densité critique est de $10^{21}$ électrons/cm$^3$). Le »claquage optique« réalisé par l'impulsion lumineuse provoque donc l'apparition de son propre écran.

La forte convergence du faisceau est, elle aussi un facteur de sécurité:

a)   vers la rétine, la divergence post focale assure un étalement de la faible proportion de lumière transmise, tel que la densité surfacique de ce rayonnement devient non dangereuse;

b)   cette forte convergence assure, avant le point focal un étalement suffisant du faisceau pour le rendre non dangereux pour les surfaces transparentes, situées avant le point à détruire et traversées.

On notera que le système cornéocristallinien intervient également comme élément convergent sur le trajet du faisceau, de sorte que ce dernier ne peut en aucun cas converger sur la rétine, le point le plus éloigné de focalisation étant distant de plus de trois millimètres de celle-ci.

L'appareil comporte en outre avantageusement une cuve de verre à faces parallèles contenant une solution d'absorbant saturable 'KODAK 9740 ®« et située sur le trajet du faisceau, après l'étalon de PEROT FABRY. Cette cuve de verre située à l'extérieur de l'oscillateur laser doit être désalignée par rapport au faisceau lui-même. L'objectif de cette cuve est de rendre difficile et impossible l'opération du laser entre la cible et le hublot de la cuve. L'absence de cette cuve entraîne la possibilité pour le laser de travailler en fonctionnement libre »free running« entre le hublot de la vuce 1 et la cible sur laquelle le faisceau est focalisé. Dans ces conditions, le laser fonctionne en régime millisecondes et n'est pas efficace. La présence de cette cuve est indispensable au bon fonctionnement opératoire du laser »mode locking«.

Sur la figure 2 on a représenté un second montage d'un appareil selon l'invention.

Sur un banc d'optique (24) proté par un châssis (22) et sous un carter (23) sont disposés la source laser (2) contenant de préférence comme élément actif un barreau de YAG (grenat double d'yttrium et d'aluminium) placé entre une cuve (1) contenant un miroir sphérique (1a) et rempli d'une solution d'absorbant saturable »KODAK 9740 ®« et un étalon PEROT-FABRY (3) qui ferme la cavité et permet de choisir la durée des impulsions. Un obturateur (4) commandé par la pédale (13) permet le déclenchement du laser. A l'extérieur de la cavité résonnante au-delà de l'étalon de PEROT-FABRY sur le trajet du faisceau est disposée une cuve à faces parallèles (15) contenant une solution d'absorbant saturable. Comme il a été dit précédemment, la présence de cette cuve à faces parallèles est nécessaire à un fonctionnement convenable du laser en mode bloqué. A la sortie de la cuve (15) est placé un dispositif optique (16) composé de lentilles et destiné à augmenter le diamètre du faisceau de façon à ce que la densité superficielle du flux lumineux dans un plan de section droite dudit faisceau soit inférieure au seuil qui provoque l'endommagement des miroirs métalliques classiques. Cette disposition permet d'utiliser des miroirs métalliques au lieu de miroirs multidiélectriques qui seraient exigés par un faisceau plus concentré. Le système optique multiplie le diamètre dudit faisceau par un facteur 20 environ.

Le faisceau à la sortie du dispositif (16) se réfléchit sur un miroir multidiélectrique (21), miroir qui le renvoie sur un miroir métallique (21a) d'où il pénètre dans le bras (9) et est acheminé par réflexion jusque sur l'optique de concentration (11). Un laser d'alignement Hélium-néon (5) est disposé de façon à traverser la cuve 1 et de suivre un trajet colinéaire à celui du faisceau laser opératoire. Dans l'obturateur (4), une lame (4a) permet le passage du faisceau rouge émis par le laser Hélium-néon alors qu'elle interdit en temps normal le passage du rayonnement infrarouge émis par le laser (2). Ce laser (2) fonctionne avec, pour milieu actif, un barreau de YAG dopé au néodyme. Le laser (2) émet, à une longueur d'onde de 10 645 Å, des impulsions se présentant sous la forme d'une rafale ou d'un train de neuf à cinq pics d'une durée unitaire de 20 à 30 microsecondes séparés par des intervalles de 6 nanosecondes. L'énergie contenue dans ce train de pics à la sortie de l'oscillateur laser est de 10 à 15 millijoules. Ce type d'émission dite en régime de blocage de modes de l'oscillateur est dû à la présence, dans la cavité (1), d'absorbant saturable »KODAK 9740 ®«, ce produit circulant en permanence devant l'un des miroirs. Le faisceau laser (5) permet sans danger pour l'opérateur d'effectuer l'alignement du laser lui-même. Par contre, il est souhaitable de disposer d'un laser d'alignement pour l'opération ophtalmologique proprement dite. On a représenté, sur la figure, ce second laser (17) dont le faisceau passe à travers un dispositif dilatateur (18) avant de se réfléchir sur les miroirs (19, 20 et 21a). Ce faisceau rouge a la propriété de passer à travers le miroir multidiélectrique (21) de façon à retrouver le trajet du faisceau laser opératoire. Le dispositif (18) permet de modifier la divergence du faisceau rouge de façon à faire coïncider au point d'impact le point focal rouge avec celui de l'infrarouge. A l'arrivée au sommet de la lampe à fente (10) placée à la sortie du bras creux (9) le miroir diélectrique (21b) réfléchissant pour l'infrarouge et transparent pour le visible permet de laisser passer dans l'axe de la lampe à fente la lumière blanche permettant d'éclairer le champ opératoire à traiter et de limiter la puissance du faisceau rouge de repérage à une valeur inférieure au seuil de danger défini par les normes internationales. Enfin, il permet de conserver intégralement l'énergie infrarouge nécessaire au traitement.

Exemples de mise en œuvre de l'appareil

Les traitements expérimentaux réalisés sur patients humains ont porté sur quatre types de cas.

### 1) Cataractes congénitales

Sur six cas de cataracte congénitale, l'âge des patients allant de 8 à 13 ans, les cristallins étant parfaitement blancs, cinq étaient des cataractes molles et une, peu épaisse, mais dure.

Dans le cas de la cataracte dure, il a été possible, en deux séances de 250 à 300 impacts (7 minutes environ) de dégager largement l'axe visuel, restituant au malade une vision de l'ordre de 1/10 de loin et $P_8$ de près sur l'échelle de Parinaud, résultat prévisible après 12 ans d'amblyopie mais néanmoins très satisfaisant.

Dans les autres cas, malgré l'absence absolue de pigment coloré, il a été possible d'ouvrier, par impacts ponctuels jointifs, la capsule cristalline antérieure.

Les masses se sont résorbées spontanément en une semaine, réouvrant largement l'axe visuel et restituant une vision allant de 3 à 7/10° de loin.

Aucune lésion de l'hyaloïde n'est apparue, ni réaction anaphylactique ou inflammatoire.

### 2) Cataractes traumatiques

Deux cas de cataractes traumatiques ont été traités, cataractes parfaitement exemptes de pigment coloré.

Cinq à six séances de 200 à 250 impacts étalées sur une semaine ont permis de dégager l'axe visuel avec pour conséquence une acuité de 6 à 9/10° avec correction.

Tous les cas pré-cités avaient, avant traitement une acuité réduite à une perception de la lumière.

### 3) Cataractes secondaires ou membranules

Dix neuf cas de cataractes secondaires ont été traités. Ces membranules sont consécutives à l'opacification de la capsule postérieure du cristallin après opération extracapsulaire de la cataracte.

Treize cas portaient sur des membranules blanches opaques, sans pigment qui ont pu être ouvertes en une à trois séances de 250 à 300 impacts.

Cinq cas concernaient des membranules translucides tendues qui limitaient l'acuité à 1/10° au mieux. Dans ces cas une séance de 5 à 450 impacts a toujours été suffisante pour restituer au patient une acuité de 8/10° à 12/10'' avec correction adaptée.

L'ouverture par impacts jointifs selon deux axes perpendiculaires semble la plus aisée puisque la traction exercée contribue au dégagement de l'axe.

Le dernier cas est celui d'une membranule de cataracte secondaire située derrière un cristallin artificiel (implant intra-oculaire). Dans ce cas l'ouverture circulaire de la membranule a été possible à travers l'implant. Les marques survenues accidentellement sur cet implant lors de mouvements incontrôlés du globe n'ont, en aucune manière, gêné la bonne acuité obtenue (7/10°).

### 4) Abord du vitré

L'intervention intra-capsulaire de cataracte exige l'ouverture ultérieure de l'hyaloïde vitréenne transparente.

Sur quatre cas traités, quatre ouvertures de cette membrane transparente ont été effectuées avec succès.

Les brides virtéennes sont des brides ou des bandes de vitré coagulé, elles sont translucides ou blanches et leur existence entraîne, par la traction qu'elles exercent, un risque de décollement de la rétine où elles sont implantées. Elles peuvent aussi, dans certains cas, maintenir soulevés des clapets de déchirure rétinienne.

Sur six cas traités, il a toujours été possible de couper ces bandes ou brides, soit directement, soit à travers un verre à trois miroirs de type Goldman. A nouveau et dans tous les cas, la sec tion a été obtenue par impacts ponctuels jointifs.

Dans l'état actuel de cet appareil, les caractéristiques temporelles de l'irradiation (rafale de plusieurs impulsions ultrabrèves) ont pour conséquence un effet de retour vers l'opérateur de l'action de destruction sur une distance d'environ 2 mm. Il est donc nécessaire, pour l'instant de respecter une distance de sécurité de 3 mm entre la cible et toute surface placée en amont à ne pas léser.

Il va de soi qui des modifications peuvent être apportées aux modes de réalisation qui viennent d'être décrits, notamment par substitution de moyens techniques équivalents, sans sortir pour cela du cadre de la présente invention.

## Revendications

1. Appareil de chirurgie ophtalmologique à laser pour la coupe de tissus biologiques, y compris de tissus transparents comportant un oscillateur laser constitué par une cuve réfléchissante (1), un barreau de grenat double d'yttrium et d'aluminium dopé au néodyme (2), un étalon de Perot-Fabry (3), des moyens d'acheminement du faisceau sur le tissu à couper, une lampe à fente (10) d'ophtalmologie, des moyens de focalisation (11) placés à la sortie de la lampe à fente, caractérisé en ce que ledit laser fonctionne en régime dit de blocage de mode et produit un faisceau à au moins une impulsion dont la densité de puissance est supérieure à $10^{12}$ Watts/$cm^2$ et dont la durée est comprise entre 20 et 400 picosecondes, lesdites impulsions se présentant sous la forme d'un train de 5 à 9 pics dont l'énergie est de l'ordre de 10 à 15 millijoules à la sortie de l'oscillateur laser (2) et en ce que le fonctionnement correct en blocage de mode est assuré au moment de l'utilisation par la disposition après l'étalon de Perot-Fabry (3), d'une cuve de verre (15) à faces parallèles contenant une solution d'absorbant saturable, sur le trajet du faisceau à l'extérieur de la cavité résonnante associée à la source laser opératoire.

2. Appareil selon la revendication 1, caractérisé en ce que l'oscillateur laser émet un train d'impulsions à une longueur d'onde de 10 645 Å.

3. Appareil selon la revendication 1 ou 2, caractérisé en ce que les moyens de focalisation (11) sont formés d'un système optique placé à la sortie de la lampe à fente (10), aligné avec le passage du faisceau de l'oscillateur laser entre ledit oscillateur et le tissu à couper.

4. Appareil selon la revendication 1, 2 ou 3, caractérisé en ce que l'oscillateur laser précité est placé entre une cuve (1) réfléchissante contenant une solution d'absorbant saturable et un étalon, un obturateur déclencheur (4) étant placé entre le barreau (2) et la cuve (1) réfléchissante de manière à ce que la cavité résonnante ainsi formée émette un train d'impulsions de 5 à 9 pics d'une durée de 20 à 30 picosecondes espacées de 6 nanosecondes.

5. Appareil selon la revendication 1, 2 ou 3, caractérisé en ce que le système optique de focalisation (11) consiste en un doublet achromatique convergent placé sur l'axe de la lampe à fente et dont la puissance est de l'ordre de 10 dioptries.

6. Appareil selon la revendication 1, 2 ou 3, caractérisé en ce qu'un système optique (16) composé de lentilles augmentant le diamètre de faisceau est disposé entre l'oscillateur laser et la lampe à fente précitée.

7. Appareil selon l'une quelconque des revendications précédentes, caractérisé en ce qu'une cellule à cristal (8) est disposée à la sortie de la source laser de manière à réaliser une conversion de longueur d'onde.

8. Appareil selon l'une quelconque des revendications précédentes, caractérisé en ce qu'une source laser (5) auxiliaire émet des radiations visibles suivant un chemin optique analogue à celui de faisceau opératoire de façon à permettre l'alignement dudit faisceau opératoire.

9. Appareil selon l'une quelconque des revendications précédentes, caractérisé en ce qu'une lame de verre absorbant à la longueur d'onde du laser opératoire mais laissant passer les radiations émises par le laser auxiliaire est disposée dans l'obturateur déclencheur (4).

10. Appareil selon l'une quelconque des revendications précédentes, caractérisé en ce qu'un verre à trois miroirs est interposé entre la sortie de l'appareil et le tissu à couper.

11. Appareil selon l'une quelconque des revendications précédentes, caractérisé en ce que les moyens d'acheminement précités consistent en un bras creux (9) comprenant un ensemble de miroirs métalliques dont l'entrée est disposée parallèlement à la direction du faisceau laser opératoire et dont la sortie est perpendiculaire à la direction du tissu à couper vers laquelle le faisceau est renvoyé par un miroir (21b) semi-transparent monté à l'intérieur de la lampe à fente d'ophtalmologie.

12. Appareil selon l'une quelconque des revendications précédentes, caractérisé en ce qu'un second laser d'alignement (17) est disposé parallèlement à l'oscillateur laser, le faisceau dudit second laser étant rendu colinéaire du faisceau de l'oscillateur laser par un jeu de miroirs.

## Patentansprüche

1. Gerät für die Augenchirurgie mittels Laser zum Durchschneiden von biologischem Gewebe, darunter auch transparentem Gewebe, enthaltend einen Laseroszillator, der aus einer reflektierenden Küvette (1), einem Stab (2) aus mit Neodym dotiertem Yttrium/Aluminium-Doppelgranat, einem Pero-Fabry-Etalon (3), Mitteln zur Übermittlung des Lichtbündels auf das zu durchschneidende Gewebe, einer ophtalmologischen Spaltlampe (10) sowie aus am Ausgang der Spaltlampe angeordneten Mitteln (11) zur Fokussierung besteht, dadurch gekennzeichnet, daß diesr Laser nach dem Prinzip der Modenblockierung arbeitet und ein Lichtbündel mit mindestens einem Impuls erzeugt, dessen Leistungsdichte bei über $10^{12}$ Watt/cm$^2$ liegt und dessen Dauer 20 bis 400 Picosekunden beträgt, wobei diese Impulse die Gestalt eines Zuges mit 5 bis 9 peaks haben, dessen Energie von der Größenordnung von 10 bis 15 Millijoules am Ausgang des Laseroszillators (2) ist, und daß das einwandfreie Arbeiten hinsichtlich der Modenblockierung im Zeitpunkt des Betriebes dadurch gewährleistet ist, daß nach dem Perot-Fabry-Etalon (3) eine Glasküvette (15) mit planparallelen Flächen, die eine Absorptionslösung mit Sättigungscharakteristik enthält, in dem Lichtweg an der Außenseite des mit dem Operationslaser verbundenen Hohlraumresonators angeordnet ist.

2. Gerät nach Anspruch 1, dadurch gekennzeichnet, daß der Laseroszillator einen Impulszug bei einer Wellenlänge von 10 645 Å emittiert.

3. Gerät nach Anspruch 1 oder 2, dadurch gekennzeichnet, daß die Fokussierungsmittel (11) gebildet sind aus einem optischen System, welches am Ausgang der Spaltlampe (10) und im Weg des Lichtbündels aus dem Laseroszillator zwischen diesem Oszillator und dem zu durchschneidenden Gewebe angeordnet ist.

4. Gerät nach einem der Ansprüche 1, 2 oder 3, dadurch gekennzeichnet, daß dieser Laseroszillator zwischen einer eine Absorptionslösung mit Sättiungscharakteristik enthaltenden, reflektierenden Küvette (1) und einem Etalon angeordnet ist, wobei ein Auslöserverschluß (4) zwischen dem Stab (2) und der reflektierenden Küvette (1) derart angeordnet ist, daß der so gebildete Hohlraumresonator einen Impulszug von 5 bis 9 peaks von einer Dauer von 20 bis 30 Picosekunden, getrennt durch 6 Nanosekunden, emittiert.

5. Gerät nach einem der Ansprüche 1, 2 oder 3, dadurch gekennzeichnet, daß dieses optische Fokussierungssystem (11) aus einem konvergierenden Doppelachromat besteht, der in der optischen Achse der Spaltlampe angeordnet ist und eine Brechkraft in der Größenordnung von 10 Dioptrien hat.

6. Gerät nach Anspruch 1, dadurch gekennzeichnet, daß ein aus Linsen bestehendes optisches System (16), welches den Durchmesser des Lichtbündels aufweitet, zwischen dem Laseroszillator und dieser Spaltlampe angeordnet ist.

7. Gerät nach einem der vorangegangenen Ansprüche, dadurch gekennzeichnet, daß am Ausgang der Laserquelle eine Kristallzelle (8) angeordnet ist, um eine Wellenlängenänderung zu realisieren.

8. Gerät nach einem der vorangegangenen Ansprüche, dadurch gekennzeichnet, daß eine Hilfslaserquelle (5) sichtbares Licht entlang eines optischen Weges emittiert, der analog ist dem Weg des Operationslichtbündels, so daß eine Ausrichtung dieses Operationslichtbündels möglich ist.

9. Gerät nach einem der vorangegangenen Ansprüche, dadurch gekennzeichnet, daß eine Glasscheibe, die bei der Wellenlänge des Operationslasers absorbiert, jedoch für die vom Hilfslaser emittierte Strahlung durchlässig ist, in dem genannten Auslöserverschluß (4) angeordnet ist.

10. Gerät nach einem der vorangegangenen Ansprüche, dadurch gekennzeichnet, daß ein Glas mit drei Spiegeln zwischen dem Ausgang des Gerätes und dem zu durchschneidenden Gewebe angeordnet ist.

11. Gerät nach einem der vorangegangenen Ansprüche, dadurch gekennzeichnet, daß diese Mittel zur Übermittlung des Lichtbündels aus einem eine Mehrzahl von Metallspiegeln aufweisenden Hohlarm (9) bestehen, dessen Eingang parallel zur Richtung des Operationslaserbündels und dessen Ausgang senkrecht zur Richtung des zu durchschneidenden Gewebes ausgerichtet ist, auf welches dieses Bündel über einen halbdurchlässigen, an der Spaltlampe angeordneten Spiegel (21b) gerichtet ist.

12. Gerät nach einem der vorangegangenen Ansprüche, dadurch gekennzeichnet, daß ein zweiter Justierlaser parallel zum Operationslaser angeordnet ist, dessen Lichtbündel mittels eines Satzes Spiegel kolinear zum Operationslichtbündel gemacht ist.

**Claims**

1. Apparatus for ophthalmological surgery using a laser, for cutting biological tissues, including transparent tissues, and comprising a laser oscillator formed by a reflecting dish (1), an yttrium and aluminium double garnet rod doped with neodymium (2), a Perot-Fabry gauge (3), means for guiding the beam on the tissue to be cut, an ophthalmological slit lamp (10), and focusing means (11) arranged at the outlet of the slit lamp, characterised in that the said laser functions in so-called mode-locking state and produces a beam having at least one pulse the power density of which is greater than $10^{12}$ Watts/cm$^2$ and the duration of which is from 20 to 400 picoseconds, the said pulses being in the form of a sequence of from 5 to 9 peaks the energy of which is of the order of from 10 to 15 millijoules at the outlet of the laser oscillator (2), and that correct mode-locking functioning is ensured at the moment of use by the arrangement, downstream of the Perot-Fabry gaube (3), of a glass dish (15), which has parallel faces and contains a solution of saturable absorbent, in the path of the beam outside the resonant cavity associated with the operating laser source.

2. Apparatus according to claim 1, characterised in that the laser oscillator emits a sequence of pulses at a wavelength of 10 645 Å.

3. Apparatus according to claim 1 or 2, characterised in that the focusing means (11) are formed by an optical system which is arranged at the outlet of the slit lamp (10) and is aligned with the path of the laser oscillator beam between the said oscillator and the tissue to be cut.

4. Apparatus according to claim 1, 2 or 3, characterised in that the above-mentioned laser oscillator is arranged between a reflecting dish (1), containing a solution of saturable absorbent, and a gauge, a Q-switch (4) being arranged between the rod (2) and the reflecting dish (1) so that the resonant cavity thus formed emits a sequence of pulses having from 5 to 9 peaks and a duration of from 20 to 30 picoseconds and being spaced apart by 6 nanoseconds.

5. Apparatus according to claim 1, 2 or 3, characterised in that the optical focusing system (11) comprises a convergent achromatic doublet which is arranged on the axis of the slit lamp and the power of which is of the order of 10 dioptres.

6. Apparatus according to claim 1, 2 or 3, characterised in that an optical system (16) composed of lenses increasing the diameter of the beam is arranged between the laser oscillator and the above-mentioned slit lamp.

7. Apparatus according to any one of the preceding claims, characterised in that a crystal cell (8) is arranged at the otulet of the laser source in such a manner as to bring about a conversion of the wavelength.

8. Apparatus according to any one of the preceding claims, characterised in that an auxiliary laser source (5) emits visible rays following an optical path analogous to that of the operating beam in such a manner as to permit the alignment of the said operating beam.

9. Apparatus according to any one of the preceding claims, characterised in that a glass plate which absorbs at the wavelength of the operating laser but allows the passage of the rays emitted by the auxiliary laser is arranged in the Q-switch (4).

10. Apparatus according to any one of the preceding claims, characterised in that a glass having three mirrors is interposed between the outlet of the apparatus and the tissue to be cut.

11. Apparatus according to any one of the preceding claims, characterised in that the above-mentioned guide means comprise a hollow arm (9) which has a set of metallic mirrors and the inlet of which is arranged parallel to the direction of the operating laser beam and the outlet of which is perpendicular to the direction of the tissue to be cut towards which direction the beam is reflected by a semi-transparent mirror (21b) mounted inside the ophthalmological slit lamp.

12. Apparatus according to any one of the preceding claims, characterised in that a second alignment laser (17) is arranged parallel to the laser oscillator, the beam of the said second laser being rendered colinear with the laser oscillator beam by a set of mirrors.

FIG.1

0 007 256

FIG.2